# EUROPEAN PATENT SPECIFICATION

(11) **EP 2 087 139 B1**
(45) Date of publication and mention of the grant of the patent: **04.01.2017**
(21) Application number: 07861764.4
(22) Date of filing: 07.11.2007
(51) Int. Cl.: C12Q 1/68, C07H 21/04

(54) **GENE EXPRESSION PROFILING BASED IDENTIFICATION OF GENOMIC SIGNATURES OF HIGH-RISK MULTIPLE MYELOMA**
AUF GENEXPRESSIONSPROFILIERUNG BASIERENDE IDENTIFIZIERUNG GENOMISCHER SIGNATUREN VON HOCHGEFÄHRLICHEM MULTIPLEM MYELOM
IDENTIFICATION FONDEE SUR LA DETERMINATION DU PROFIL D'EXPRESSION GENETIQUE DE SIGNATURES GENOMIQUES DE MYELOMES MULTIPLES A HAUT RISQUE

(30) Priority: 07.11.2006 US 857456 P
(43) Date of publication of application: 12.08.2009
(73) Proprietor: The Board of Trustees of the University of Arkansas, Little Rock, AR 72207-3608 (US)
(72) Inventor: SHAUGHNESSY, John D., Roland, AR 72135 (US); ZHAN, Fenghuang, Little Rock, AZ 72211 (US); BARLOGIE, Bart, Little Rock, AR 72205 (US); BURLINGTON, Bart E., Brisbane, CA 94005 (US)
(74) Representative: Heaton, Joanne Marie
(86) International application number: PCT/US2007/023404
(87) International publication number: WO 2008/057545

(56) References cited:
- WO-A2-2005/116259
- US-A1- 2005 112 630
- US-A1- 2005 260 664
- ZHAN FENGHUANG ET AL: "The molecular classification of multiple myeloma" BLOOD, AMERICAN SOCIETY OF HEMATOLOGY, US, vol. 108, no. 6, 1 September 2006 (2006-09-01), pages 2020-2028, XP002444356 ISSN: 0006-4971
- ZHAN FENGHUANG ET AL: "DNA amplification and over-expressionof CKS1B is inversely correlated with p27 levels and associated with aggressive clinical course in multiple myeloma (MM)" PROCEEDINGS OF THE ANNUAL MEETING OF THE AMERICAN ASSOCIATION FOR CANCER RESEARCH, NEW YORK, NY, US, vol. 46, 1 April 2005 (2005-04-01), page 638, XP001525125 ISSN: 0197-016X
- SHAUGHNESSY JOHN D JR ET AL: "Using genomics to identify high-risk myeloma after autologous stem cell transplantation." BIOLOGY OF BLOOD AND MARROW TRANSPLANTATION : JOURNAL OF THE AMERICAN SOCIETY FOR BLOOD AND MARROW TRANSPLANTATION JAN 2006, vol. 12, no. 1 Suppl 1, January 2006 (2006-01), pages 77-80, XP24918008 ISSN: 1083-8791
- ZHAN F ET AL: "Global gene expression profiling of multiple myeloma, monoclonal gammopathy of undetermined significance, and normal bone marrow plasma cells" BLOOD, AMERICAN SOCIETY OF HEMATOLOGY, US, vol. 99, no. 5, 1 January 2002 (2002-01-01), pages 1745-1757, XP003024165 ISSN: 0006-4971
- SHAUGHNESSY JOHN D JR ET AL: "A validated gene expression signature of high risk multiple myeloma is defined by deregulated expression of genes mapping to chromosome" BLOOD, vol. 108, no. 11, Part 1, November 2006 (2006-11), page 37A, XP009129102 & 48TH ANNUAL MEETING OF THE AMERICAN-SOCIETY-OF-HEMATOLOGY; ORLANDO, FL, USA; DECEMBER 09 -12, 2006 ISSN: 0006-4971
- ZHAN ET AL.: 'The molecular classification of multiple myeloma' BLOOD vol. 108, no. 6, 15 September 2006, pages 2020 - 2028, XP002444356

## Description

### BACKGROUND OF THE INVENTION

### Field of the Invention

The present invention generally relates to the field of cancer research. More specifically, the present invention relates to the use of gene expression profiling to identify genomic signatures specific for high-risk multiple myeloma useful for predicting clinical outcome and survival.

### Description of the Related Art

Multiple myeloma (MM), a malignancy of terminally differentiated plasma cells homing to and expanding in the bone marrow, is characterized by a tremendous heterogeneity in outcome following standard and high dose therapies. Although, many of the genetic and molecular lesions associated with disease initiation are known, the lesions that promote an aggressive clinical course have remained elusive.

All myelomas can be broadly divided into hyperdiploid and non-hyperdiploid disease [1-4]. Hyperdiploidy, typically associated with trisomies of chromosomes 3, 5, 9, 11, 15, 19 and 21, is present in approximately 60% of patients [5]. Unsupervised clustering and non-negative matrix factorization of high resolution oliogonucelotide array comparative genomic hybridization (aCGH) data has revealed that hyperdiploid myeloma can be further segregated into two groups, one exhibiting trisomies of the odd chromosomes described above and another exhibiting, in addition, gains of chromosomes 1q and 7, deletion of chromosome 13 and absence of trisomy 11 [6]. Non-hyperdiploid myeloma can also be divided into two groups, one characterized by high-level amplification of chromosome 1q and deletions of chromosomes 1p and 13, and another characterized by absence of chromosome 1 abnormalities but harboring deletions of chromosomes 8 and 13 [6]. Furthermore, transcriptional activation of *CCND1*, *CCND3, MAF, MAFB, or FGFR3*/*MMSET* (resulting from translocations involving the immunoglobulin heavy chain locus on chromosome 14q32) is typical of non-hyperdiploid myeloma and present in approximately 40% of cases [5,7,8].

Using unsupervised hierarchical clustering of global gene expression patterns, subgroups exhibiting strong correlations with hyperdiploidy and recurrent translocations were recently defined and validated the existence of seven myelomas [9]. Two high-risk entities were identified, one revealing over-expression of proliferation genes and derived from cases evolving from the other six classes, while the other was defined by the t(4;14)(p16;q32) [9].

Gains of the long arm of chromosome 1 (1q) are one of the most common genetic abnormalities in myeloma [10]. Tandem duplications and jumping segmental duplications of the chromosome 1q band, resulting from decondensation of pericentromeric heterochromatin, are frequently associated with disease progression [11-13]. Using aCGH on DNA isolated from plasma cells derived from patients with smoldering myeloma, Rosinol and colleagues showed that the risk of conversion to overt disease was linked to gains of 1q21 and loss of chromosome 13 [14]. These findings were confirmed by using interphase fluorescence in situ hybridization (FISH) analysis. Additionally, it was demonstrated that gains of 1q21 acquired in symptomatic myeloma were linked to inferior survival and were further amplified at disease relapse [15].

WO 2005/116259 describes a list of 70 genes whose expression may be analyzed to determine a survival prognosis after treatment for cancer.

Shaughnessy et al [Biology of Blood and Marrow Transplantation 12:77-80(2006)] describes that expression of *CKS1B* is linked with survival outcome following treatment with bortezomib and thalidomide.

Zhan et al [Blood 2006 108:6 2020-2028] describes the use of gene profiling assays using a small number of genes for predicting survival in multiple myeloma in patients treated with melphalan [9].

### SUMMARY OF THE INVENTION

The present invention is directed to a method of gene expression profiling to identify genomic signatures linked to a prognosis of survival in a subject with symptomatic multiple myeloma or multiple myeloma, comprising determining the expression levels of genes consisting of *KIF14, SLC19A1, CKS1B, YWHAZ, MPHOSPH1, TMPO, NADK, LARS2, TBRG4, AIM2, ASPM, AHCYL1, CTBS, MCLC, LTBP1* and genes detectable with nucleic acids having the Affymetrix reference numbers 242488_at and 1557277-9_at on a U133PLW2.0 Affymetrix microarray in a nucleic acid sample from plasma cells isolated from the subject, and determining the presence of abnormal expression levels, thereby prognosing the subject.

### BRIEF DESCRIPTION OF THE DRAWINGS

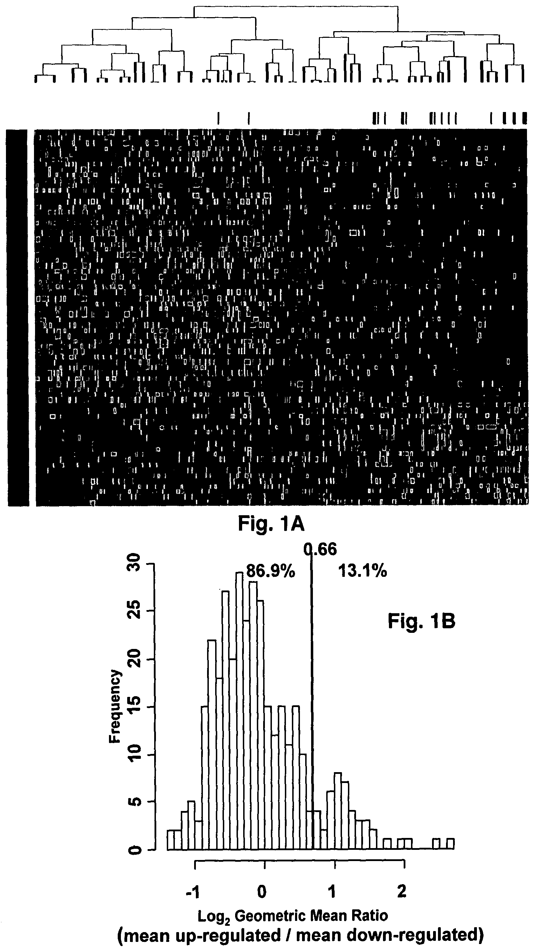

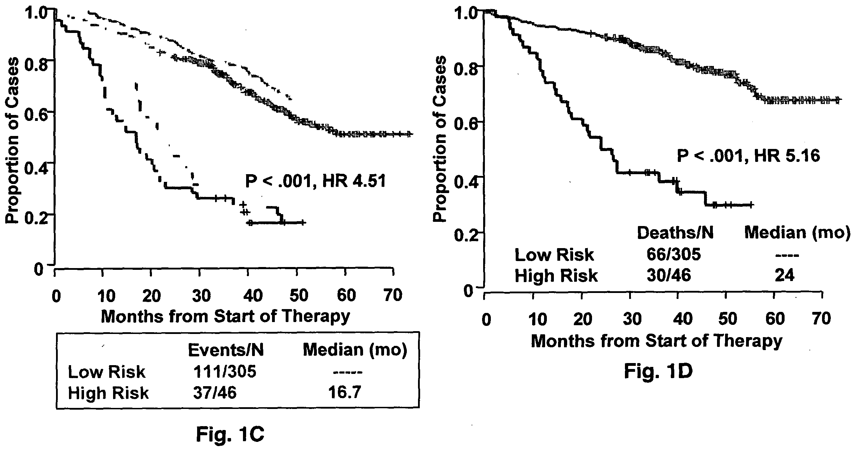

**Figures 1A-1D** show gene expression patterns that distinguish risk groups in training cohort. **Figure 1A** shows heat maps of the 70 genes that illustrate remarkably similar expression patterns among 351 newly diagnosed patients used to identify the 70 genes. Red bars above the patient columns denote cases with disease-related deaths. The 51 genes in rows designated by the red bar on the left (top rows, up-regulated), identified patients in the upper quartile of expression at high risk for early disease-related death. The 19 gene rows designated by the green bar (down-regulated), identified patients in the lower quartile of expression at high risk of early disease-related death. **Figure 1B** shows training cohort frequencies for sample differences between ratios of the mean of log₂ expression of the 51 up-regulated genes /19 down-regulated genes. This self-normalizing expression ratio has a marked bimodal distribution, consistent with the upper/lower quartile log rank differential expression analysis, which was designed to detect genes that define a single high-risk group (13.1%) with an extreme expression distribution. Interpreted as an up/down-regulation ratio on the log₂ scale, higher values are associated with poor outcome. The vertical line shows the high- v low-risk cutoff for the log₂-scale ratio determined by K-means clustering: the percentage of samples below and above the cutoff is also shown. **Figures 1C**-**1D** demonstrates Kaplan-Meier estimates of event-free (**Figure 1C**) and overall survival (**Figure 1D****)** in low-risk myeloma (blue) and high-risk myeloma (red) showed inferior 5-yr actuarial probabilities of event-free survival (18% v 60%, P <.0001; HR = 4.51) and overall survival (28% v 78%, P <.0001, HR = 5.16) in the 13.1% patients with high-risk signature.

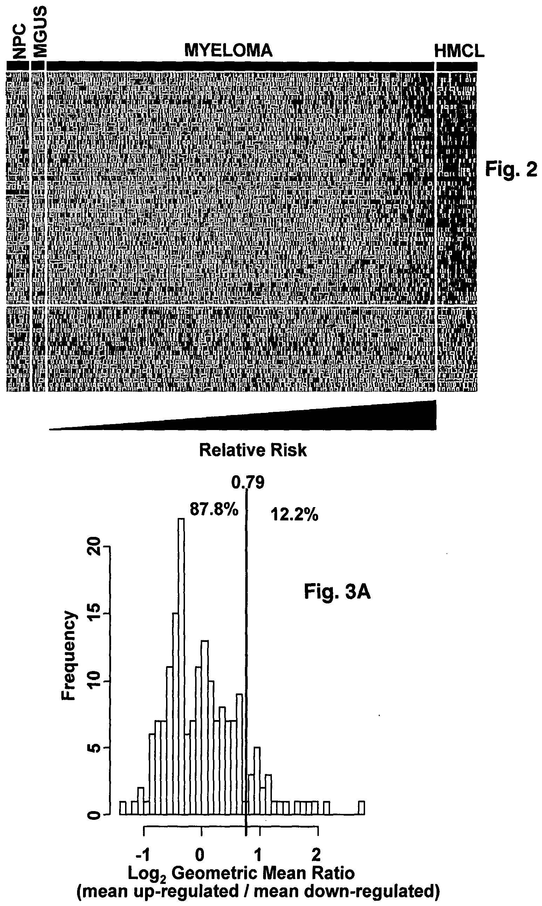

**Figure 2** shows gene expression clustergram of 70 high-risk genes in plasma cells from 22 healthy subjects (NPC), 14 subjects with monoclonal gammopathy of undetermined significance (MGUS), 351 patients with newly diagnosed myeloma (MM) and 42 human multiple myeloma cell lines (HMCL). Each row represents a gene and each column a sample. The genes are ordered from top to bottom based on the rank in Table 1. Red color for a gene indicates expression above the median and blue color below the median. Samples within myeloma risk groups were ordered so that the predicted risk increases continuously from left to right.

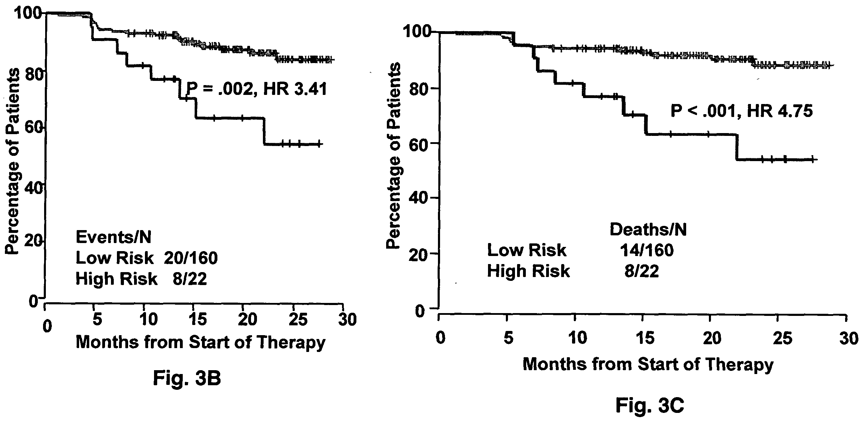

**Figures 3A-3C** show risk group distribution and survival analyses in the test cohort. **Figure 3A** demonstrates test cohort frequencies for the ratio of the mean of the log₂ up/down-regulated genes. The cutoff for high-risk was determined by independent clustering of the log₂ ratio. The training and validation sets have a similar distribution for this expression summary of the 70 genes, including similar cutoffs for high-risk and similar proportions clustered into the high-risk group. **Figures 3B-3C** show Kaplan-Meier estimates of (**Figures 3B**) event-free and (**Figures 3C**) overall survival between molecular risk groups in the test cohort.

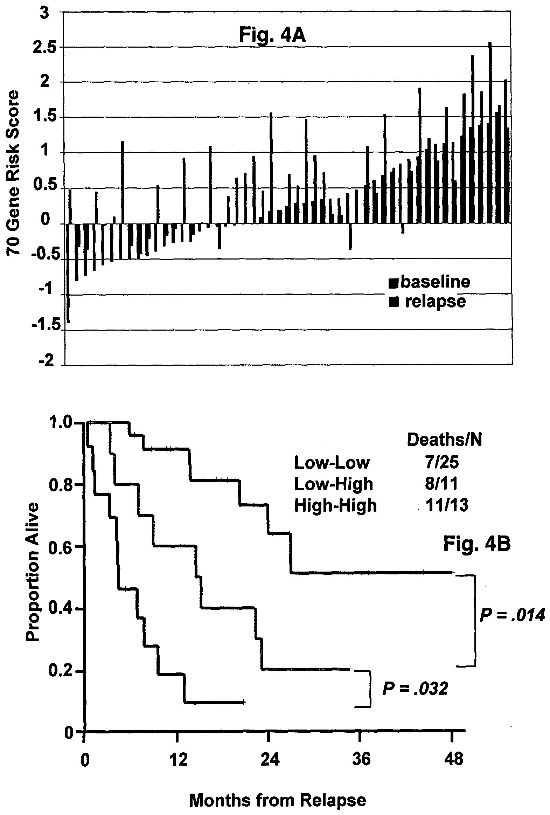

**Figures 4A-4B** show that 70-Gene risk score at diagnosis and relapse predicts post-relapse survival. **Figure 4A** shows 70-gene risk score in paired diagnostic (blue) and relapse (red) samples of 51 cases from the training cohort. The gene expression risk score is indicated to the left. Sample pairs are order from left to right based on lowest baseline score. **Figures 4B** shows Kaplan-Meier plots of post-relapse survival of the three groups defined by low-risk both at diagnosis and relapse (Low-Low), low-risk at diagnosis and high-risk at relapse (Low-High) and high risk at both time points (High-High).

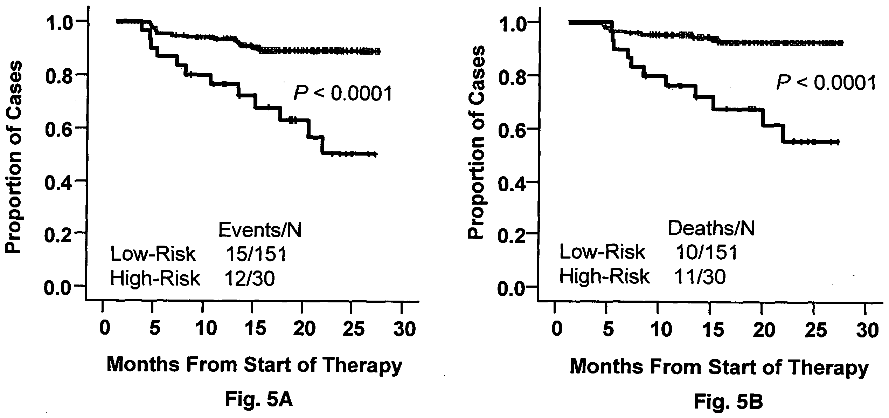

**Figures 5A-5B** show event-free and overall survival in risk groups defined by the 17-gene model in the test set. The 181 newly diagnosed MM cases were predicted into high-risk (16.6%) and low-risk (83.4%) groups. Kaplan-Meier estimates of survival in low-risk and high-risk myeloma showed 2-year actuarial probabilities of event-free survival (**Figure 5A**) of 88% for the high risk (red) versus 50% for low risk (blue) (P < .0001) and overall survival (**Figure 5B**) of 91% for the high-risk (red) versus 54% for the low-risk (blue) (P <.0001).

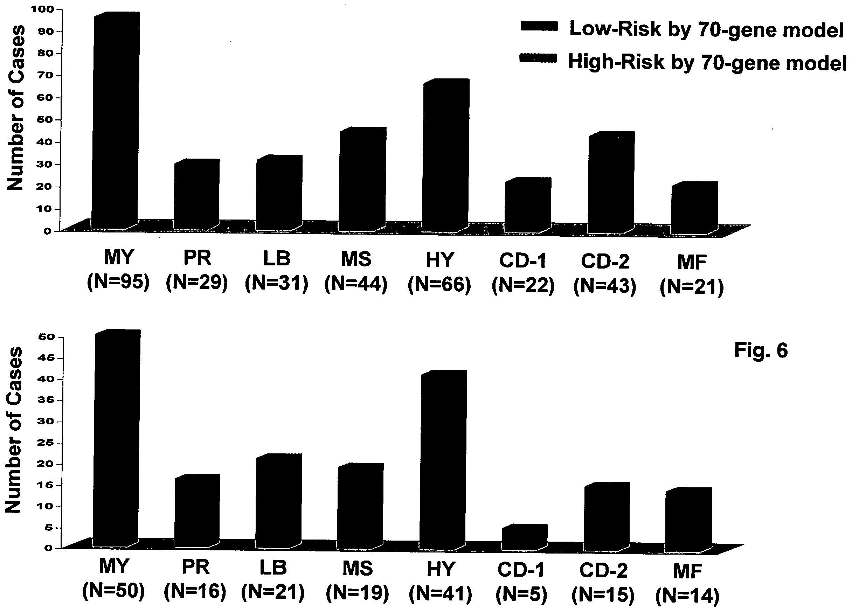

**Figure 6** shows relationship between high- and low-risk defined by the 70-gene supervised model and the 7-subgroup unsupervised classifier (9). Data are presented as a stacked bar-view of the number of high-risk (red) and low-risk cases (blue) in each of the 7 subtypes, including the group of cases with the so-called myeloid signature (MY) (far left).

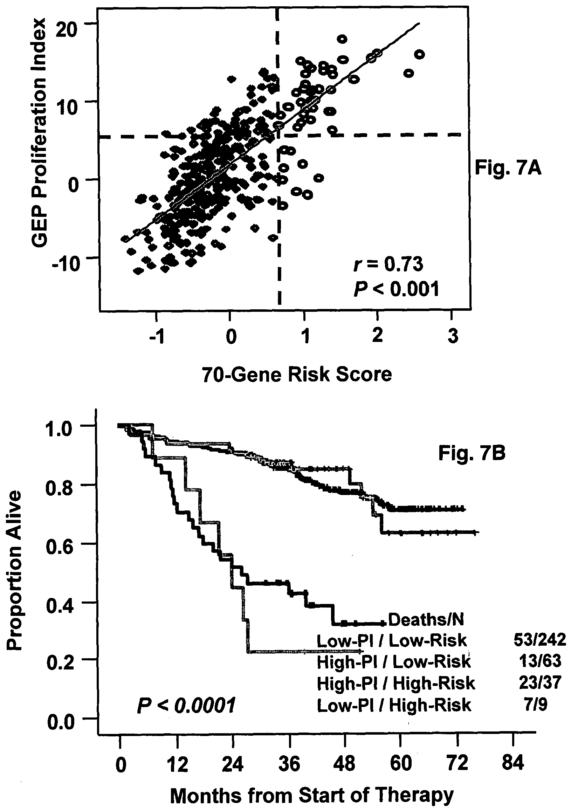

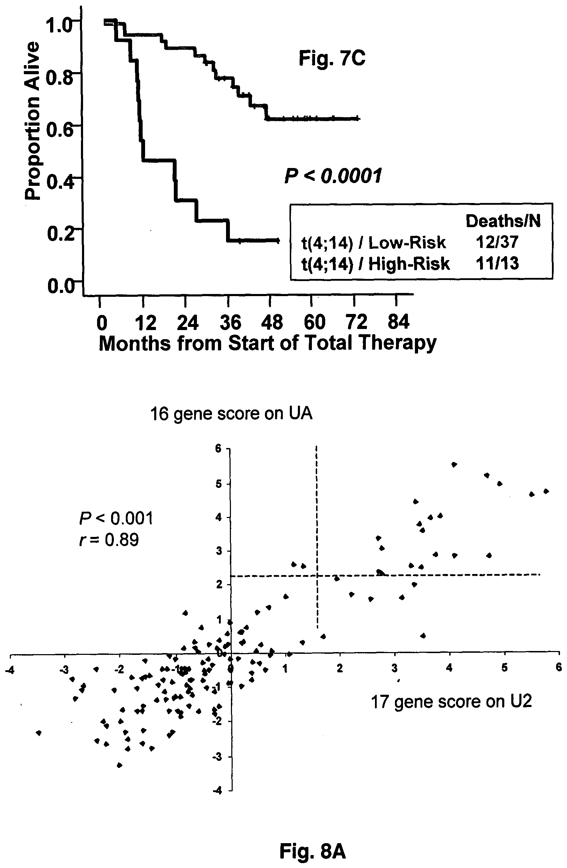

**Figures 7A-7C** related the 70 gene model-defined high-risk with molecular features. **Figure 7A** shows a scatter plot of gene expression-based proliferation index (x-axis) by 70-gene risk score in 351 cases of the training cohort. Low-risk cases (blue) and high-risk cases (red) defined by the 70-gene model (see text) are indicated. The two variables show a substantial degree of correlation (r = 0.73; P < 0.001). To evaluate the influence of the two variables on outcome, the population was divided into 4 subgroups using a PI of cut point of 5 and a high-risk cut-point of .66. The groups are defined by the intersection of the two green lines. The upper left quadrant contains cases with high PI/low-risk, the upper right quadrant cases with high PI/high-risk, the lower left quadrant contains cases with low PI/low-risk and the lower right quadrant contains cases with low PI/high-risk. The line represents the linear trend in the data. **Figure 7B** shows Kaplan-Meier plots of overall survival estimates of the four groups defined in **Figure 7A****,** revealing no impact of PI within risk groups. **Figure 7C** shows Kaplan-Meier plots of overall survival estimates of t(4;14)-positive myeloma in relationship to the 70-gene high-risk score designation of the given sample, showing the profound impact of high- and low-risk scores.

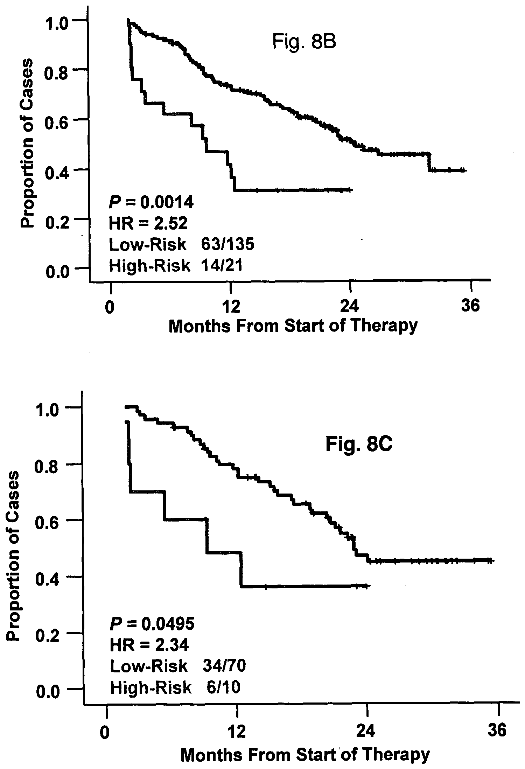

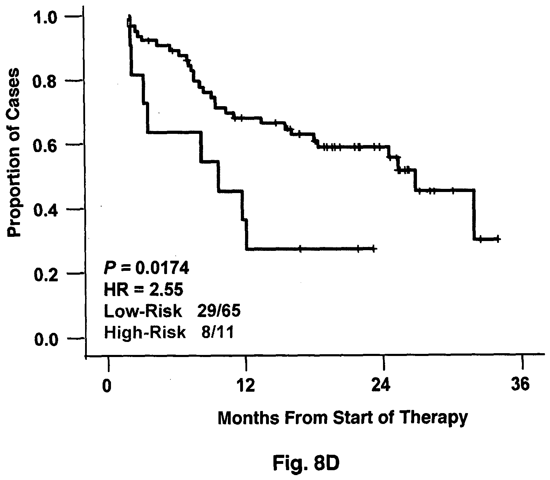

**Figures 8A-8D** show that the 17-gene expression model can be used to predict outcome in relapsed disease treated with single agent Bortezomib. **Figure 8A** shows prediction of Risk groups using U2 and U4 data and the 17-gene and 16 gene models. The Risk group prediction analysis was performed on 144 newly diagnosed cases of myeloma. There was a high degree of correlation between the two models. **Figure 8B** shows the Kaplan Meier survival analysis in the Millennium data set. The 16-gene model was applied to 156 relapsed myeloma patients treated in the APEX trial [31]. 13.5% and 86.5% were predicted as high-risk and low-risk, respectively. Overall survival estimates of one-year actuarial probabilities were 74% for the low risk disease (blue) versus 32% for high-risk (red) (P=0.0014; HR=2.52). **Figure 8C** shows overall survival in the bortezomib cohort. Kaplan-Meier estimates of survival in the low-risk and high-risk myeloma showed one-year actuarial probabilities of OS of 79% for low-risk disease versus 36% for high risk (*P*=0.0495; HR=2.34). **Figure 8D** shows overall survival in the dexamethasone cohort. Kaplan Meier estimates of survival in low-risk and high-risk myeloma showed one-year actuarial probabilities of OS of 64% for the low-risk disease versus 23% for high risk (P=0.0174; HR=2.55). Analyses of the entire APEX trial (669 patients) revealed superior OS with bortezomib vs dex, despite patient cross-over (30 vs 20 months; P=0.027; 22 month median follow up, 44% events occurred) [32].

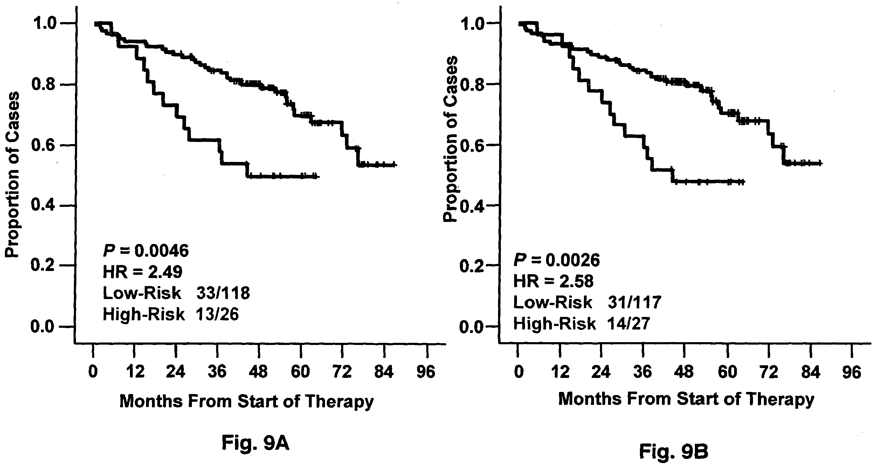

**Figures 9A-9B** show overall survival analysis in the 144 newly diagnosed cases with both UA and U2. **Figure 9A** shows the 17-gene U2-derived model applied to 144 newly diagnosed myeloma patients. Kaplan-Meier estimates of overall survival revealed significant differences in the 5-year actuarial probabilities of survival (50% vs 68%, P=0.0046; HR=2.40) in low-risk myeloma (blue) and high-risk myeloma (red), respectively. **Figure 9B** shows Kaplan-Meier estimates of OS in the 144 patients based on risk as defined by the 16-gene UA-derived model. It revealed 5-year actuarial probabilities of survival at 46 vs 70%, *P*=0.0026; HZ=2.58 in high- versus low-risk, respectively.

### DETAILED DESCRIPTION OF THE INVENTION

The survival variability of patients with multiple myeloma is not well accounted for with current laboratory parameters, such as beta-2-microglobulin and albumin levels employed in the ISS staging system [23]. De-novo high-risk disease may be fundamentally different from myeloma acquiring drug resistance and an aggressive clinical course after recurrent relapses.

The marked increase in the frequency of high-risk designation from 13% at diagnosis to 76% at relapse provides molecular evidence of disease evolution that influences post-relapse outcome. An aggressive myeloma phenotype, whether de novo or acquired, may develop through a similar mechanism. With further refinement of the model discussed herein, the present invention contemplates developing tools for quantitative risk assessment during the entire course of therapeutic management.

In addition to its clinical relevance, the findings presented herein may also shed important light on the underlying molecular mechanisms that drive disease progression.
A striking feature of the high-risk signature was the significant over-representation of genes from chromosome 1: nearly 50% of 19 under-expressed genes and 30% of 51 over-expressed genes were derived from chromosome 1p and 1q, respectively. The predominance of chromosome 1q-derived genes in the high-risk score is in agreement with a recent report showing that disease progression is associated not only with an increase in copy number but also the percentage of cells with 1q21 amplification [15]. The gene expression-based high-risk signature defined herein is also remarkably consistent with a class of disease defined by high resolution aCGH profiling, characterized by high-level amplification of 1q21 and deletion of 1p13 [6]. Taken together, these data suggest that alterations in this chromosome, either through genetic and/or epigenetic modifications, may play a significant role in disease evolution by providing a growth and/or survival advantage.

Using a combination of high-resolution aCGH and microarray profiling, 47 minimal common regions (MCRs) of genomic gain across the myeloma genome and 207 genes mapping within these MCRs whose expression increases with increased in copy number were identified [6]. When the expression of these copy number-sensitive genes was compared between the high- and low-risk classes defined by the 70-gene model, genes mapping to MCRs at 1q21, 1q22 and 1q43-q44 were found to be significantly over-expressed in high-risk disease.

Although chromosome 1 genes are implicated as key players in disease progression, the residence of 4 other genes, *FABP5, YWHAZ EXOSC4,* and *EIFC2,* in the 8q21-8q24 region implies that gains of 8q may also contribute to high-risk disease. These genes, encompassing recently defined MCRs of gain/amplification at 8q24.12-8q24.13 and 8q24.2-8q24.3 [6]. Interestingly, expression of *MYC,* mapping to a MCR at 8q24, was not linked to survival in the current study.

Chromosome 13q14 deletion is an important predictor of survival in patients with myeloma treated on tandem transplant trials [24]. It is noteworthy that loss of expression of a single gene mapping to chromosome 13q14, *RFP2,* previously identified as a candidate tumor suppressor gene in B-CLL with significant homology to *BRCA1*[25] was again linked to poor survival in this analysis. *RFP2* was also found to exhibit copy number-sensitive expression in myeloma [6]. The frequent amplification of chromosome 1 in many late stage cancers, including 1q21 in non-Hodgkin's lymphoma, Wilm's tumor, Ewing's sarcoma, breast and ovarian cancer, [12,26-30] warrants studies to determine whether the gene expression model described here has prognostic relevance in other cancers. Although the present invention has used the gene expression profiling to identify genomic signatures of multiple myeloma, the method and kit provided herein may be used to identify same genes or different genes that are predictive of outcome in other cancers. For the same reason, the genes identified herein may be predictive of outcome in other cancers.

Through multivariate discriminant analyses, of the original 70 genes, 17 probe sets could be used to detect high-risk myeloma. Hence, present invention contemplates developing and validating a quantitative RT-PCR-based assay that combines these staging/risk-associated genes with molecular subtype/etiology-linked genes identified in the unsupervised molecular classification. Assessment of the expression levels of these genes may provide a simple and powerful molecular-based prognostic test that would eliminate the need for testing so many of the standard variables currently in use with limited prognostic implications devoid also on drug-able targets. Use of a PCR-based methodology would not only dramatically reduce time and effort expended in fluorescence in-situ hybridization-based analyses but also reduce markedly the quantity of tissue required for analysis. If these gene signatures are unique to myeloma tumor cells, such test may be useful after treatment to assess minimal residual disease, possibly using peripheral blood as a sample source.

Furthermore, the present invention also applied the 17-gene model to predict outcome in relapsed disease treated with single agent, Bortezomib. As this model was originally developed for myeloma patients treated with multi-agent chemotherapy followed by transplant supported HDT and maintenance, the result discussed herein was somewhat unexpected. These data strongly suggest that outcome related gene expression patterns are similar in relapsed and newly diagnosed MM patients. The high-risk gene expression model also appeared to be independent of the specific therapeutic modality, suggesting that it identifies patients that are generally insensitive to currently employed drugs or drug combinations. This interpretation is supported by the observation that the previously validated survival classifier developed with a subset of bortezomib treated relapsed myeloma patients [34] also identified high-risk patients treated with Total Therapy 2 (data not shown).

Beyond differences in microarray platform, various other characteristics of the study design distinguish these 2 MM clinicogenomic datasets. Notably, the Millennium samples were obtained at multiple centers as opposed to a single center in the UAMS study. UAMS purified plasma cells by CD138-based immuomagnetic bead selection, while the 96 participating clinical centers in the Millennium study each performed a negative selection method before shipping frozen tumor specimens.

Two important implications follow from these observations. First, as varied gene expression patterns often represent distinct underlying biological states of normal [35] and transformed tissues [35, 36, 37], it seems likely that the high risk signature is related to a biological phenotype of drug resistance and/or rapid relapse in multiple myeloma. Accordingly, this myeloma phenotype deserves further study in order to better characterize the most relevant pathways and identify therapeutic opportunities. The relatively large gene expression datasets employed here provide one avenue to more fully define these tumor types. Second, while some hurdles remain to routine clinical implementation of high-risk stratification, this work highlights that a specific subset of myeloma patients continues to receive minimal benefit from current therapies. A practical method to identify such patients should notably improve patient care. For patients predicted to have a favorable outcome, efforts to minimize toxicity of standard therapy might be indicated, while those predicted to have poor outcome regardless of the current therapy utilized may be considered for early administration of experimental regimens. The present invention contemplates determining if this tumor GEP model of high-risk could be implemented clinically and if it would be relevant for other front-line regimens, including those that test novel combinations of proteasome inhibitors and/or IMIDs with standard anti-myeloma agents and HDT. Finally, prior analyses of response to therapy suggested that a bortezomib response classifier was specific to single-agent bortezomib therapy [34]; additional analyses of these 2 datasets are needed to clarify the biological pathways associated with the activity of all three therapies, as well as pathways linked specifically to Total Therapy, single-agent bortezomib or single-agent dexamethasone.

In one embodiment of the present invention, there is provided a method of gene expression profiling to identify genomic signatures linked to a prognosis of survival in a subject with symptomatic multiple myeloma or multiple myeloma, comprising determining in the expression levels of genes consisting of *KIF14, SLC19A1, CKS1B, YWHAZ, MPHOSPH1, TMPO, NADK, LARS2, TBRG4, AIM2, ASPM, AHCYL1, CTBS, MCLC, LTBP1* and genes detectable with nucleic acids having the Affymetrix reference numbers 242488_at and 1557277-9_at on a U133PLW2.0 Affymetrix microarray and nucleic acid sample from plasma cells isolated from the subject, and determining the presence of abnormal expression levels, thereby prognosing the subject.

Such a method may further comprise applying a multivariate step-wise discriminant analysis (MSDA) across the genomic signatures, where the application identifies the 17 genes linked to at least one of survival or capable of discriminating high-risk and low-risk disease.

Further, a high mean ratio of expression obtained by using this method may be indicative of a genomic signature associated with high-risk disease. Such a genomic signature of high-risk disease may correlate with shorter duration of complete remissions, event free, early disease-related death or a combination thereof. Additionally, an individual bearing the genomic signature of high-risk disease may be selected for secondary prevention trials. Alternatively, a low mean ratio of expression may be indicative of a genomic signature associated with a low-risk disease. Such a genomic signature of low-risk disease may correlate with longer duration of complete remissions, longer survival, a good prognosis or a combination thereof.

Furthermore, the method described herein may predict clinical outcome and survival of an individual, may be effective in selecting treatment for an individual suffering from a disease, may predict post-treatment relapse risk and survival of an individual, may correlate molecular classification of a disease with the genomic signature defining the risk groups, or a combination thereof. The molecular classification may be CD1 and may correlate with high-risk multiple myeloma genomic signature. The CD1 classification may comprise increased expression of MMSET, MAF/MAFB, PROLIFERATION signatures or a combination thereof. Alternatively, the molecular classification may be CD2 and may correlate with low-risk multiple myeloma genomic signature. The CD2 classification may comprise HYPERDIPLOIDY, LOW BONE DISEASE, CCND1/CCND3 translocations, CD20 expression or a combination thereof. Additionally, type of disease whose genomic signature is identified using such a method may include but is not limited to symptomatic multiple myeloma or multiple myeloma.

As used herein, the term, "a" or "an" may mean one or more. As used herein in the claim(s), when used in conjunction with the word "comprising", the words "a" or "an" may mean one or more than one. As used herein "another" or "other" may mean at least a second or more of the same or different claim element or components thereof.

The following examples are given for the purpose of illustrating various embodiments of the invention and are not meant to limit the present invention in any fashion.

### EXAMPLE 1

### Patients

Purified plasma cells were obtained from normal healthy subjects and from patients with monoclonal gammopathy of undetermined significance (MGUS) and with overt myeloma requiring therapy. Patient characteristics of training (n=351) and validation groups (n=181) have been previously described.⁹ Of 351 cases in the training group, 51 also had samples taken at relapse. Both protocols utilized induction regimens, followed by melphalan-based tandem autotransplants, consolidation chemotherapy and maintenance treatment.

### EXAMPLE 2

### Gene expression profiling

Plasma cell purifications and GEP, using the Affymetrix U133Plus2.0 microarray, were performed as previously described [9,16]. Microarray data and outcome data on the 532 patients used in this study have been deposited in the NIH Gene Expression Omnibus under accession number GSE2658.

### EXAMPLE 3

### Statistical and microarray analyses

Affymetrix U133Plus2.0 micro-arrays were preprocessed using GCOS1.1 software and normalized using conventional GCOS1.1 scaling. Log rank tests for univariate association with disease-related survival were performed for each of the 54,675 'Signal' summaries. Specifically, log rank tests were performed for quartile 1 (Q1) vs. quartiles 2-4 (Q2-Q4) and quartile 4 (Q4) vs. quartiles 1-3 (Q1-Q3) in order to identify under- and over-expressed prognostic genes, respectively. A false discovery rate cut-off of 2.5% was applied to each list of log-rank P-values [17] yielding 19 under- and 51 over expressed probe sets. Heat-map-column dendrograms were computed with hierarchical clustering using Pearson's correlation distances between patient pairs' log₂-scale expression. Column-dendrogram branches were sorted left-to-right based upon each patient's difference between average log₂-out the objects and obtain the ends and advantages mentioned, as well as those objects, ends and advantages inherent herein.

### EXAMPLE 1

### Patients

Purified plasma cells were obtained from normal healthy subjects and from patients with monoclonal gammopathy of undetermined significance (MGUS) and with overt myeloma requiring therapy. Patient characteristics of training (n =351) and validation groups (n=181) have been previously described.⁹ Of 351 cases in the training group, 51 also had samples taken at relapse. Both protocols utilized induction regimens, followed by melphalan-based tandem autotransplants, consolidation chemotherapy and maintenance treatment.

### EXAMPLE 2

### Gene expression profiling

Plasma cell purifications and GEP, using the Affymetrix U133Plus2.0 microarray, were performed as previously described [9,16]. Microarray data and outcome data on the 532 patients used in this study have been deposited in the NIH Gene Expression Omnibus under accession number GSE2658.

### EXAMPLE 3

### Statistical and microarray analyses

Affymetrix U133Plus2.0 micro-arrays were preprocessed using GCOS1.1 software and normalized using conventional GCOS 1.1 scaling. Log rank tests for univariate association with disease-related survival were performed for each of the 54,675 'Signal' summaries. Specifically, log rank tests were performed for quartile 1 (Q1) vs. quartiles 2-4 (Q2-Q4) and quartile 4 (Q4) vs. quartiles 1-3 (Q1-Q3) in order to identify under- and over expressed prognostic genes, respectively. A false discovery rate cut-off of 2.5% was applied to each list of log-rank P-values [17] yielding 19 under- and 51 over expressed probe sets. Heat-map-column dendrograms were computed with hierarchical clustering using Pearson's correlation distances between patient pairs' log₂-scale expression. Column-dendrogram branches were sorted left-to-right based upon each patient's difference between average log₂-scale expression of the 51 up-regulated and the 19 down-regulated genes: this difference is interpreted as an up/down-regulated mean ratio (i.e. geometric mean) on the log₂ scale. This simple, univariate summary of the 70-gene expression profile for each patient may enhance robustness to residual array effects (i.e. after MAS5.0 processing) that increase or decrease all 70 genes multiplicatively, and is also independent of the MAS5.0 scale factor. Weighting expression by hazard ratios, unstandardized or standardized (i.e. Wald statistics), does not improve this score, and the design was to use no supervision by overall survival (OS) or event-free survival (EFS) beyond the gene-by-gene log rank tests. The log₂ up/down-regulated mean ratio was then clustered using K-means into 3 groups to separate out the small extreme right mode in the histogram: the two groups with lower up/down mean ratios were combined. The single extreme mode in the up/down mean expression ratio is consistent with the extreme quartile log rank tests used in the differential expression analysis, though the histograms and the right-hand side of the heat maps suggest that the extreme patient group is smaller than 25%, closer to 13%.

Note that different clustering algorithms and numbers of groups generate high mean ratio groups between 12% and 29% of patients: K-means (with K=3) was chosen since it was best (i.e. among simple algorithms for the univariate log₂ ratio) at separating the small right hand mode from the larger distribution. Any univariate cut-off capturing between 10% and 30% patients is significant for OS in the 351 patient training set. In the 181 patient validation set, K-means clustering was performed independently to produce an independent cutoff for high vs. low log₂ ratio. Application of the training set cutoff in the validation set provides an independently validated classification error of 1.7% (i.e. 3 patients in the low risk validation set are classified as high risk). An early validation was presented based upon an independent cohort treated under a newer protocol in order to illustrate and provide strong supporting evidence for the association of the 70 gene up/down-regulated mean ratio with overall survival. The high-risk cutoff for the mean ratio should be associated with survival broadly in newly-diagnosed patients, regardless of protocol, so that the difference in protocol for the validation set strengthens the evidence rather than weakening it. The mean ratio may also be associated with outcome in previously treated patients, however new cutoffs for the ratio would be required to define a high risk group. An important caveat is that the 70 genes are not particularly suited to explaining outcome among the lower two thirds of patients (ranked by the mean ratio): this is consistent with the original log rank screens which lumped 75% of the patients into a single group for the Q1 and Q4 log rank tests: these genes identify the most aggressive myeloma plasma cells, by design.

To determine the exact genome map location and order of the probe sets on the Affymetrix U133Plus2.0 microarray, software was developed to automatically query the NCBI search engine (http://www.ncbi.nlm.nih.gov/entrez) for all gene start and end sites. The location of each probe set was then compared with its corresponding gene or transcript start point and aligned from the p arm telomere to q arm telomere. In this manner more than 98% (53,581 of 54,675) probe sets were given an exact chromosome position. The software used for mapping can be found at (http://lambertlab.uams.edu/software).

Distributions of event-free, overall survival and duration of complete remission (dated from onset of complete response) were estimated using the Kaplan-Meier method, [18] and log rank statistics were used to test for their equality across groups [19]. Chi-square tests and Fisher's exact tests were used to test for the independence of categories. Multivariate proportional hazards analyses, adjusted the effects of predictors and the proportions of observed heterogeneity explained by the combined predictors, i.e. R², were computed [20]. Table 5 summarizes a multivariate linear-regression analysis of the log₂-scale up-/down-regulation ratio. The statistical package R version 2.0.1 [21] was used for this analysis.

A stepwise multiple linear discriminant analysis (MSDA) with the Wilk's lambda criterion, [22] was used to select a subset of the 70 genes equally capable of differentiating high-risk and low-risk MM. The MSDA selected the following equation: Discriminant score = 200 638_s_at x 0.283 - 1 557 277_a_at x 0.296 - 200 850_s_at x 0.208 + 201 897_s_at x 0.314 - 202 729_s_at x 0.287 + 203 432_at x 0.251 + 204 016_at x 0.193 + 205 235_s_at x 0.269 + 206 364_at x 0.375 + 206 513_at x 0.158 +211 576_s_at x 0.316 + 213 607_at x 0.232 - 213 628_at x 0.251 - 218 924_s_at x 0.230 - 219 918_s_at x 0.402 + 220 789_s_at x 0.191 + 242 488_at x 0.148 (where the variables represent the Affymetrix value for the particular probe). The cutoff value was 1.5, such that values less than 1.5 indicated the sample belonged to the low risk group and values greater than 1.5 indicated the sample belonged to the high risk MM group. Both forward and backward variable selections were performed. The choice to enter or remove variables was based on minimizing the within group variability with respect to the total variability across all the samples.

### EXAMPLE 4

### Gene expression patterns are an independent predictor of survival in myeloma

Toward identifying a distinctive molecular signature of high-risk myeloma, the early disease-related death was correlated with gene expression extremes. Gene expression levels from microarray data on CD138-selected plasma cells from 351 newly diagnosed patients were divided into quartiles, and log rank tests were used to identify 70 genes that were linked to short survival: 51 had high (quartile 4, Q4) and 19 had low (quartile 1, Q1) expression (**Table 1**), the expression levels of which are depicted in a colorgram (**Figure 1A**). Noteworthy is the simultaneous up-regulation of the 51 genes and down-regulation of the 19 genes among the patients on the right-hand side. The difference between the average of Q4 and Q1 log₂-scale expression for each patient was therefore calculated. This unsupervised expression summary is interpretable as a log₂-scale up- vs. down-regulated mean expression ratio (referred to as a risk score). Its frequency distribution reveals a distinct group having high log₂ up-/down-regulation ratios (**Figure 1B****).** This is precisely the kind of extreme-expression group that Q1 and Q4 log rank tests were designed to screen for, though both the frequency plot and heat map suggest that the group's size is smaller than 25%. Unsupervised K-means clustering of the log₂ ratio estimated its proportion at 13.4%. This group exhibited significantly poorer event-free survival (**Figure 1C**, P < 0.001) with an unadjusted hazard ratio (HR) of 4.51 and also inferior overall survival (**Figure 1D****,** P < 0.001) with an unadjusted HR of 5.16. Significant associations are expected for the training cohort, in whom the 70 genes were discovered, and they are reported for illustration.

The early disease-related death outcome was chosen specifically for the purpose of identifying target genes in aggressive myeloma and, consequently, only 24 deaths were available for the log rank tests used for gene discovery in the original cohort of 351 patients. Supervised clustering with the 70 genes was applied to plasma cells from 22 healthy donors, 14 cases of MGUS, 351 patients of the training cohort and 38 human myeloma cell lines. Results revealed that the low-risk myeloma group had a pattern similar to MGUS and normal plasma cells, while the high-risk group exhibits a pattern similar to human myeloma cell lines (**Figure 2**).

Next, the association of the expression signature with overall survival was examined in an independent test cohort of 181 patients. Indeed, an independent, unsupervised clustering of the log₂-scale up-/down-regulated expression ratio identified a proportionally similar subset of patients exhibiting extreme dysregulation (12.2%, **Figure 3A**). A similar result of survival distribution and hazard ratio was found in both event-free survival (HR=3.41, P = 0.002, **Figure 3B**) and overall survival (HR=4.75, P < 0.001, **Figure 3C**) as seen in the training cohort. Absence of a high-risk score identified a favorable subset of patients with a 5-yr continuous complete remission of 60% as opposed to a 3-yr rate of only 20% in those with a high-risk score (data not shown).

To further assess the validity of the clusters with respect to clinical features, correlations of various clinical parameters were analyzed between the low- and high-risk subgroups in both training (**Table 2**) and test sets (**Table 3**). A remarkable similarity of clinical feature distribution in risk groups was observed in both training and test cohorts: higher serum levels of β₂-microglobulin, C-reactive protein, creatinine and lactate dehydrogenase (LDH) as well as FISH-defined chromosome 13 deletion and metaphase cytogenetic abnormalities all were significantly more common in the high-risk group of both training and test sets (P < 0.05). Similarly, the clinically more benign *CCND1* subgroup predominated in the low-risk and the *MMSET*/*FGFR3* subgroup in the high-risk cohort, as depicted for the training set in **Table 2** and for the test set in **Table 3.**

In a multivariate analysis of variables associated with overall and event-free survival, the high up-/down-regulation ratio predictor (high risk score) retained its significance after adjustment for competing genetic and clinical variables (even including the International Staging System) in both the training set (**Table 4**: HR = 4.1, P < 0.001) and the test set (data not shown, P=0.025). Importantly, the high-risk score also was the only independent baseline parameter that affected complete response duration adversely (hazard ratio, 3.07; P < 0.001). This strong prognostic performance of the GEP-derived risk score can be partly explained by its strong association with known clinical prognostic variables, as shown by a multivariate analysis with the up-/down-regulation ratio as the outcome (**Table 5**). While the variables in Table 5 may serve as temporary, partial substitutes for a broadly available GEP assay, Table 4 suggests that such an assay, combined with high-risk translocations (also measurable via GEP), has the potential to provide a powerful simple prognostic test for myeloma.

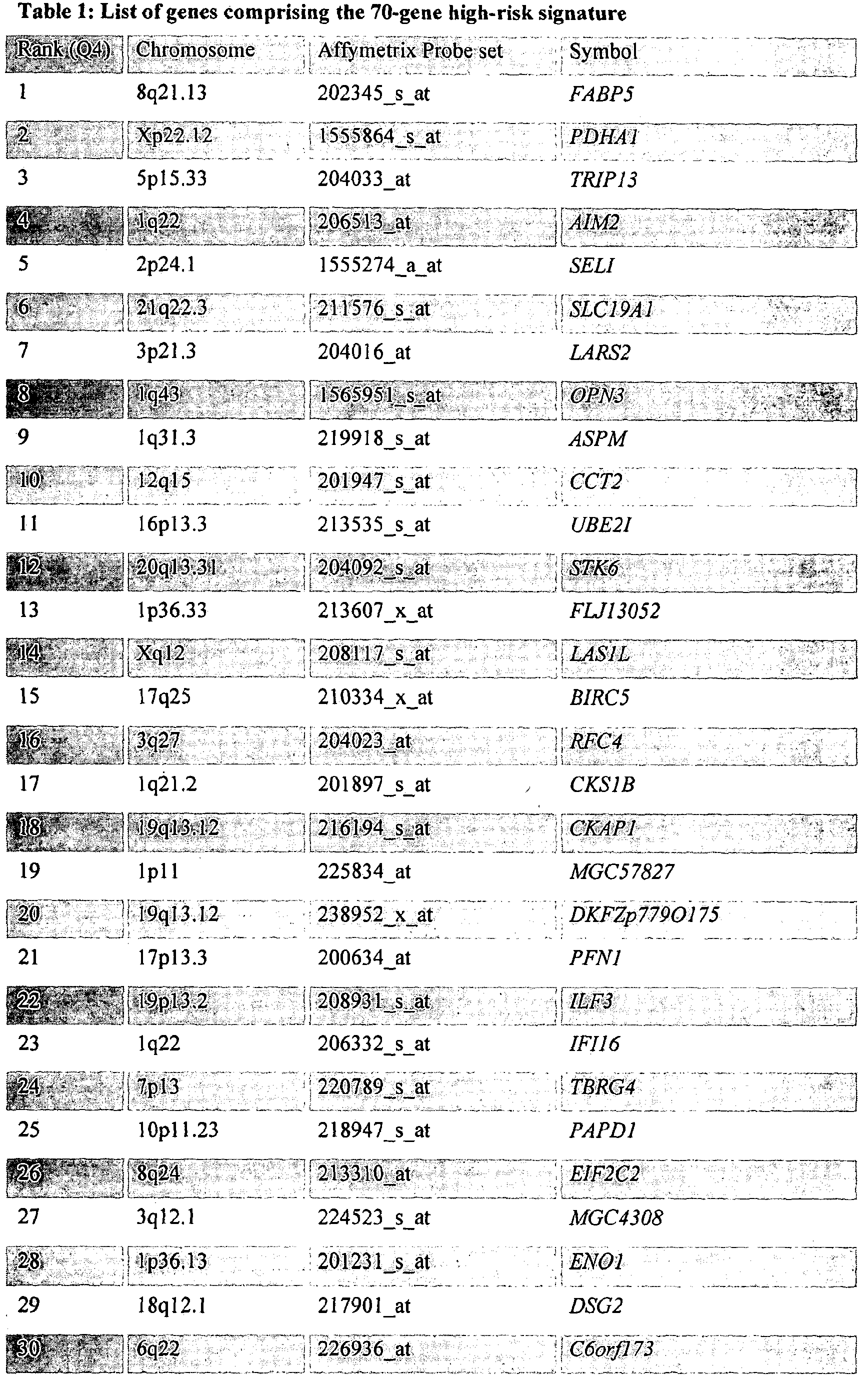

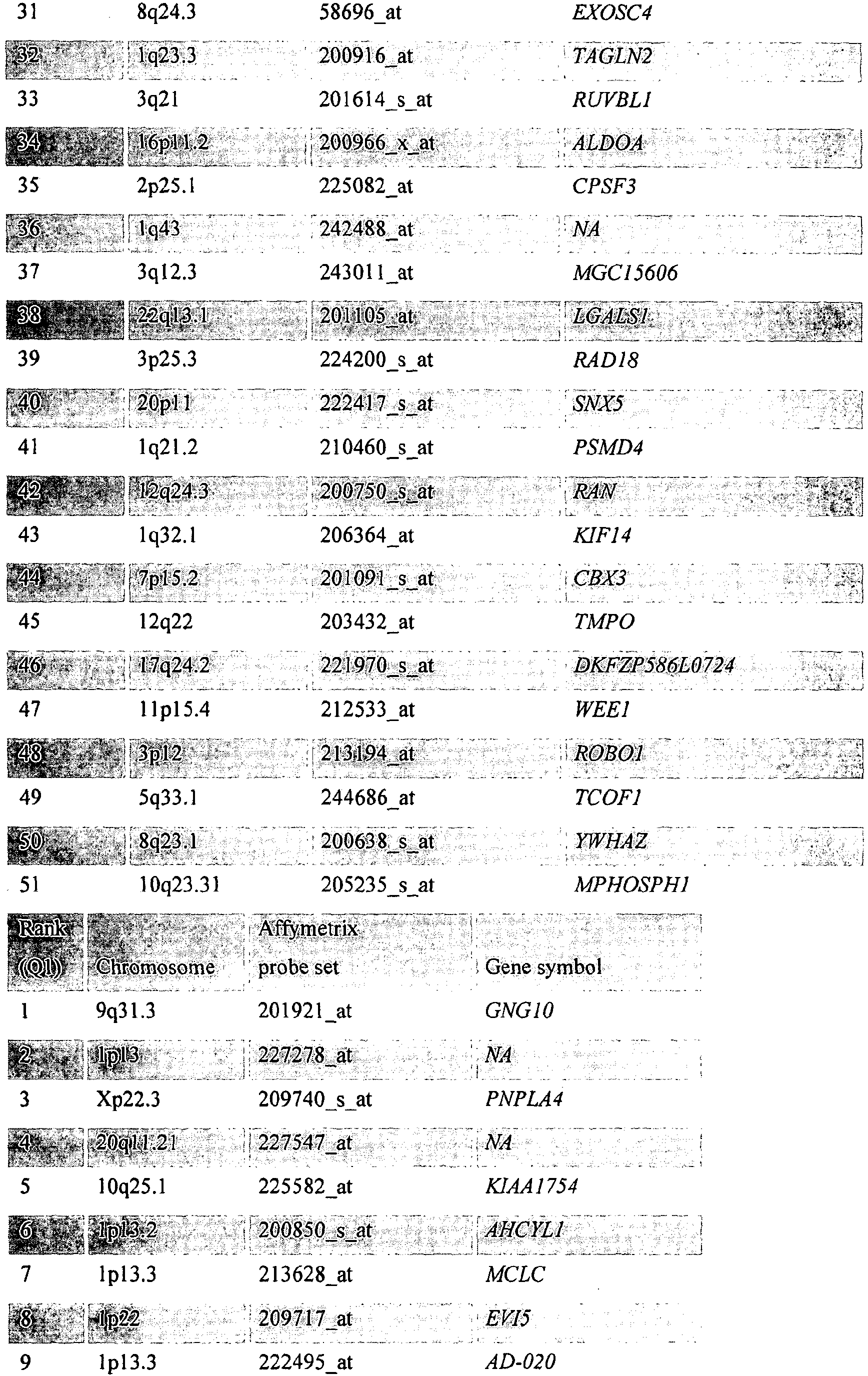

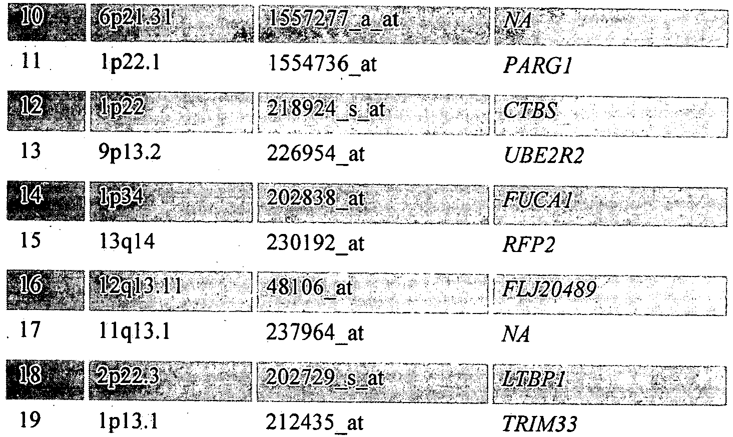

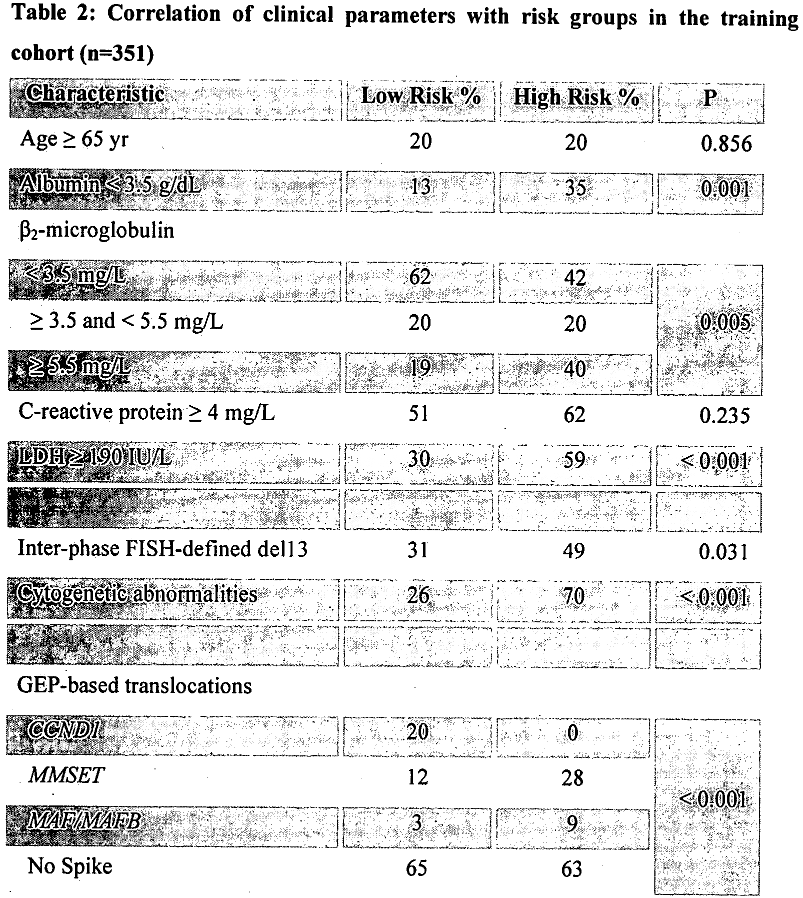

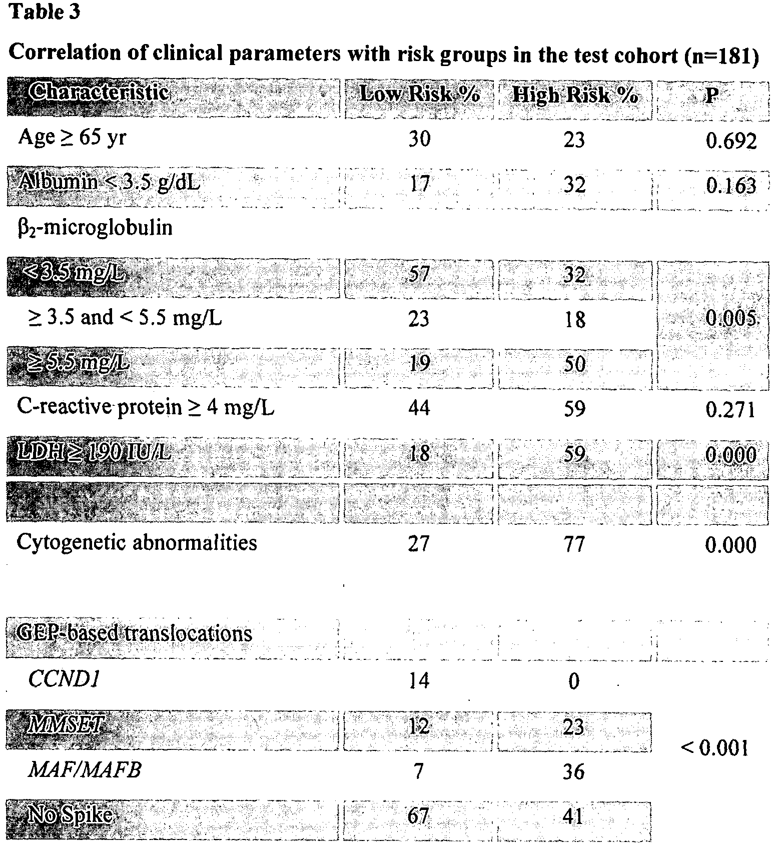

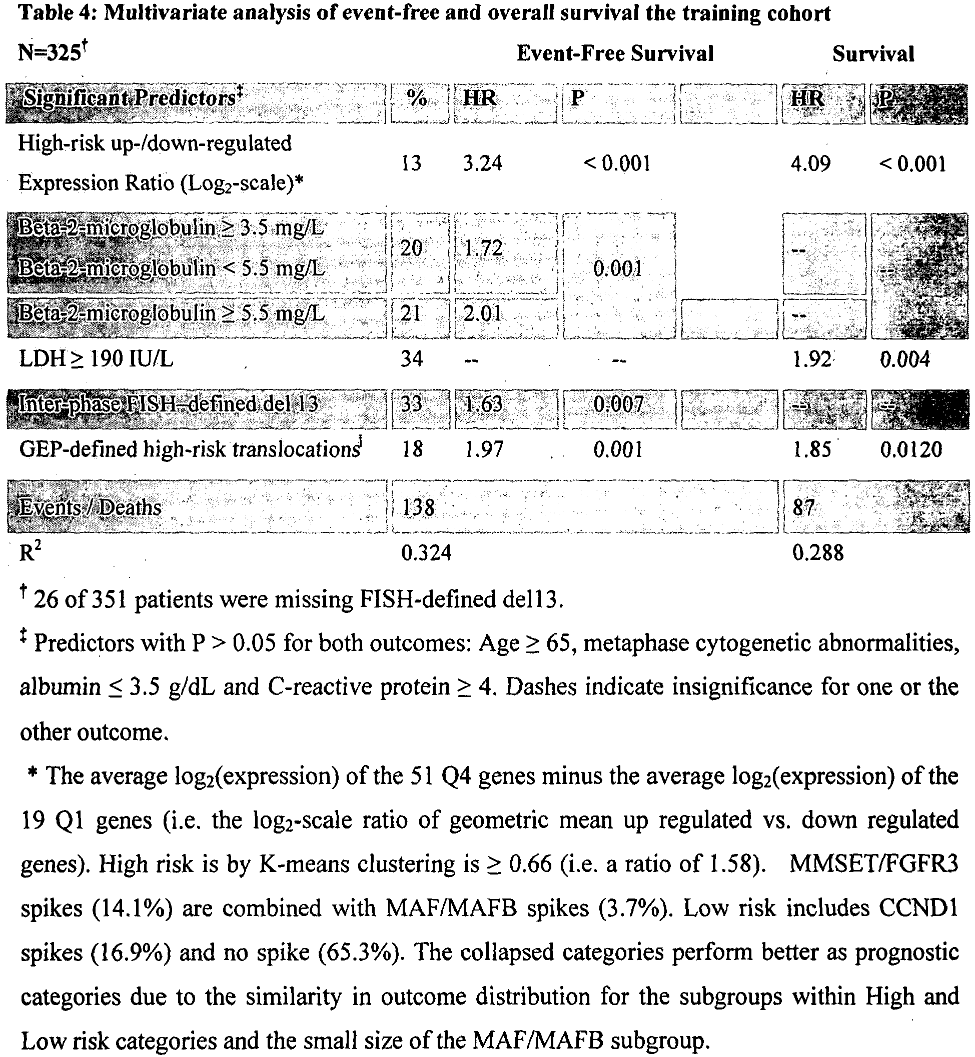

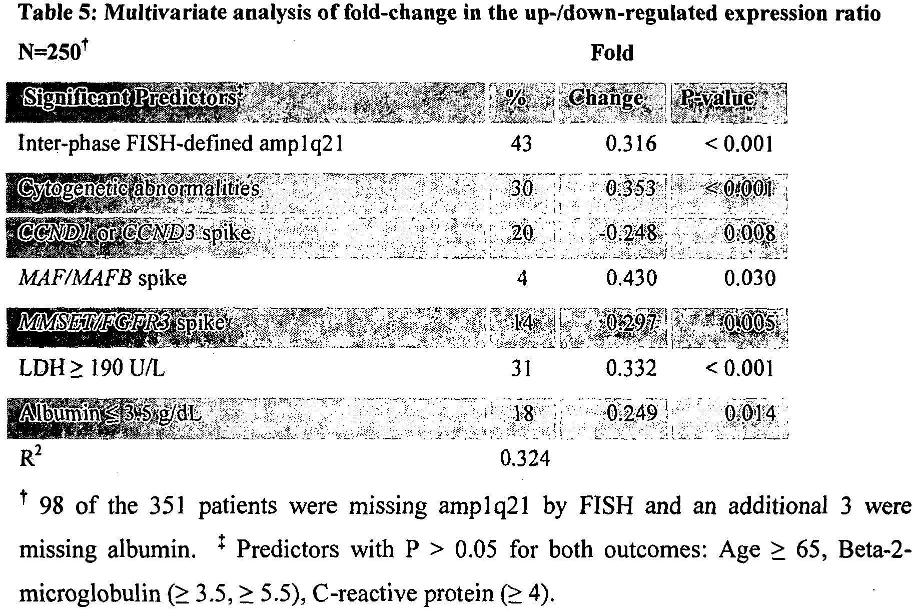

### EXAMPLE 5

### Gene-expression model predicts post-relapse risk and survival

When the 70-gene risk model was applied to relapse samples from 51 of the 351 patients of the training set, 39 (76%) exhibited a high-risk score (Figure 4A). In a paired analysis of baseline and relapse samples, the 25 patients with low-risk designation at both diagnosis and relapse had a superior post-relapse survival, followed by 11 patients with low-risk designation at diagnosis and high-risk at relapse and 13 patients exhibiting a high risk designation at both observation times (Figure 4B). There were only 2 cases with high-risk at diagnosis and low-risk at relapse.

### EXAMPLE 6

### Chromosome 1 genes are overrepresented in high risk model

To determine whether the 70-gene high-risk signature may reflect specific gains or losses of genomic DNA in high-risk MM, the map positions of the 70 genes comprising the gene expression risk signature were compared (**Table 6**). While representing only 10% of genes on the microarray, 21 (30%) of the 70 high-risk genes mapped to chromosome 1 (P < .0001): 9 of 19 (47%) quartile 1 genes mapped to 1p with 5 mapping to 1p13; among 12 of 51 (24%) quartile 4 genes mapping to chromosome 1, 9 resided on 1q while the 4 on 1p mapped to the extreme telomeric and centromeric regions of the p arm. These data suggest that gain of DNA material on 1q and loss of 1p are significant determinants of high-risk in MM.

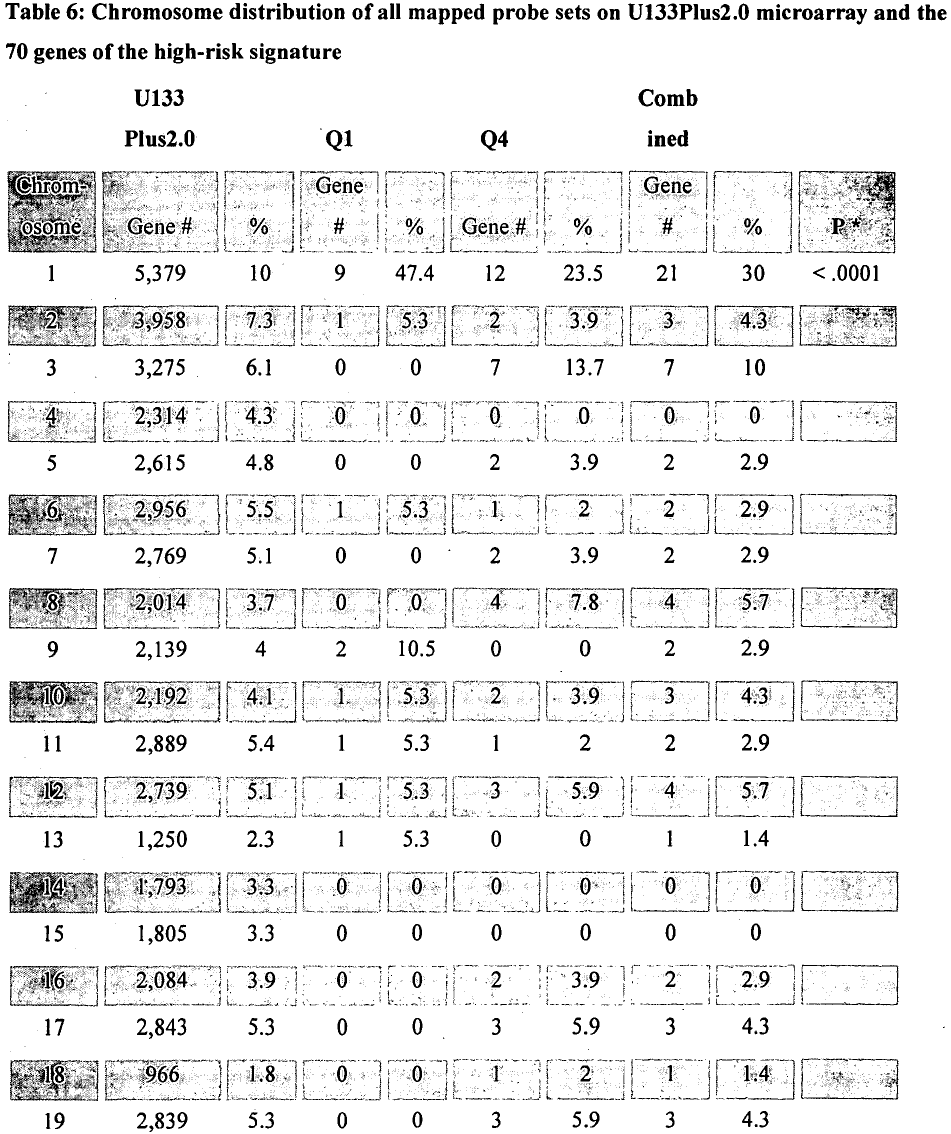

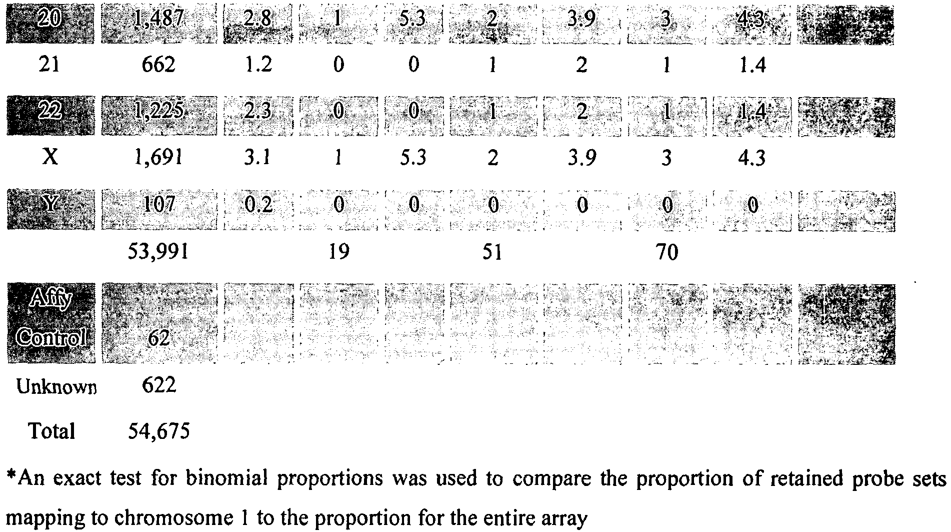

### EXAMPLE 7

### A 17-gene model can substitute for 70-gene model

Having shown that high-risk is likely related to genomic alterations of chromosome 1, a minimum set of genes capable of discriminating high-risk and low-risk myeloma was determined. Applying a multivariate step-wise discriminant analysis (MSDA) of the 70 high-risk associated genes across the high-risk (N = 46) and low-risk (N = 305) cases defined by the 70-gene model in the training set, 17 genes were identified in the resultant linear discriminant function (**Table 7**). It is noteworthy that 3 of the 5 (60%) Q1 genes and 5 of the 12 (45%) Q4 genes in the model map to 1p and 1q, respectively. The 17-gene model was then applied to the training group and predicted, with 97.7% accuracy, the correct class based on the high-/low-risk classification of the 70-gene model (**Table 8A**). A cross-validation analysis was performed where samples were removed one at a time from the sample set, and the predictive model was recalculated without that sample. Then the model was used to classify the removed observation. In this cross-validation approach, the prediction accuracy was 96.9%. The 17-gene model was then applied to the test set of 181 newly diagnosed patients receiving the second protocol UARK 03-033. The MSDA model again correctly classified 150 of 159 (94.3%) low-risk and 21 of 22 (95.5%) high-risk samples (**Table 8B**). The Kaplan-Meier estimates of overall survival of the high-risk and low-risk groups were similar whether defined by the 17-gene model (**Figure 5**) or the 70-gene model (**Figure 3D**).

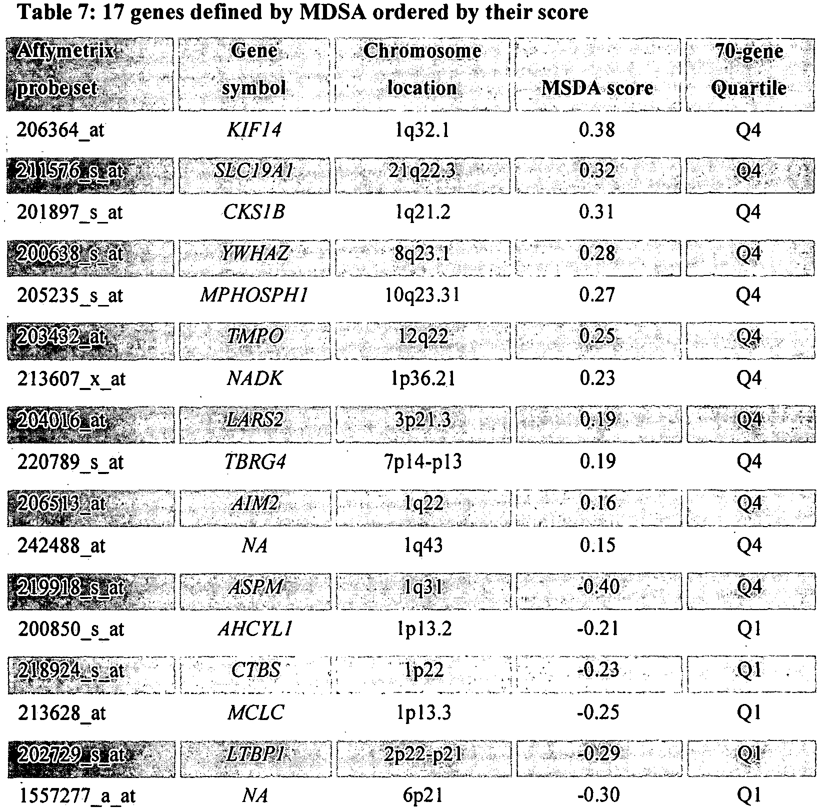

**Table 8A: Confusion matrix of risk prediction in training set using 17-gene model**

| **70-Gene Risk Group** | | **17-Gene Risk Group** | |
|---|---|---|---|
| | **Total** | **Low** | **High** |
| **Low** | 305 | 298 | 7 |
| **High** | 46 | 1 | 45 |

**Table 8B. Confusion matrix of risk prediction in test set using 17-gene model**

| **70-Gene Risk Group** | | **17-Gene Risk Group** | |
|---|---|---|---|
| | **Total** | **Low-Risk** | **High-Risk** |
| **Low** | 159 | 150 | 9 |
| **High** | 22 | 1 | 21 |

### EXAMPLE 8

### Relating_70 gene model-defined high risk myeloma with molecular subgroups defined by unsupervised hierarchical cluster analysis

The high-risk model identified was examined in the context of a previously defined molecular classification [9] High-risk disease designation pertained to all myeloma classes except for CD-2 type characterized by *CCND1* or *CCND3* spikes and *CD20* and *VPREB3* expression (**Figure 6**). Despite a strong correlation between the high-risk signature and the Proliferation (PR) subgroup (**Figure 6**), the presence of outlier cases suggests that the high-risk signature not only reflects tumor cell proliferation but may encompass also other features of disease conferring short survival such as drug resistance. Analysis of the 351 training cases according to 70-gene high-risk cut point of .66 and a proliferation index (PI) of 5 (**Figure 7A**) revealed that high and low PI designations failed to identify subgroups with different survival among low-risk and high-risk groups (**Figure 7B**). When applied to the 50 patients with t(4;14)(p16;q32), the 70-gene risk score again separated low- and high-risk subgroups (P < 0.001) (**Figure 7C**).

### EXAMPLE 9

### Applying the 17-gene model to predict outcome in relapsed disease treated with single agent, Bortezomib

To investigate whether the 17-gene model might predict high-risk in the Millennium dataset, the U133Plus2.0-derived (U-2) 17-gene model was reconstructed using U133AB (U-AB) data. Briefly, Affymetrix U133Plus2.0 (U2) microarray (Affymetrix, Santa Clara, CA) on CD138-selected plasma cells from 351 newly diagnosed cases of myeloma treated with high dose therapy and stem cell support has been described (GEO accession GSE2658) [33]. The exact same RNA sample from the first 144 of the 351 described above was also analyzed on the U133A/B (UA) microarray (Affymetrix, Santa Clara, CA) and this data has been deposited in the GEO (GSE8991). UA data on 156 patients with relapsed multiple myeloma treated with either bortezomib or dexamethasone in a phase III trial has been described^{2,3} (GEO accession number pending).

A stepwise multiple linear discriminant analysis (MSDA) with the Wilks lambda criterion using U2-derived data was used to define 17-gene model predictive of high-and low-risk disease [33]. Survival distributions were presented with the use of the Kaplan-Meier method and compared with the log-rank test. Statistical tests were performed with the software package SPSS 12.0 (SPSS, Chicago, IL).

Of the 17 genes identified using the U2 platform, 16 were on the UA microarray (**Table 9**). The multivariate stepwise discriminant analysis (MSDA) model used to develop the 17 gene U2-based model was then applied to the signal intensity of the 16 UA genes in the 144 cases that have data from both types of array. By correlating the resultant risk scores derived from the U2- and UA-derived models, this analysis revealed a strong correlation (**Figure 8A****;** r = 0.89; *P* < 0.001) and showed that a score of greater than 1.6 was high-risk in both models. Using 1.6 as a cut point in both models, a confusion matrix showed a 96.5% concordance between the U2 and UA defined high- and low-risk (**Table 10**) such that only 5 patients differed in their risk assignment (the UA version of the classifier reassigned 2 high risk and 3 low risk patients) Kaplan-Meier survival analysis of the high and low risk groups defined by the U2 model revealed a significant difference in overall survival between the two groups with an unadjusted hazard ratio (HR) of 2.49. (**Figure 9A**) Similarly, Kaplan-Meier analysis of the UA-defined risk groups revealed a significant difference in survival between the two groups (*P* = 0.0026) with high risk being associated HR of 2.58 (**Figure 9B**). These results indicate the ability to reconstruct the risk model using the UA platform.

Next, the UA version was then applied to the Millennium dataset of 156 cases of relapsed disease treated with either bortezomib or dexametheasone. The high-risk model defined 13.5% of the 156 APEX patients as high-risk by using the 16-gene model. These patients had significantly shorter survival times than the remaining patients (**Figure 8B****,** P=0.0014, HR=2.5). This result indicates that the high-risk model was relevant in relapsed multiple myeloma as well as newly diagnosed disease. This result also suggests the model is relevant for patients treated with single-agent therapies such as bortezomib and dexamethasone. Therefore, each of the APEX treatment arms (80 and 76 patients treated with bortezomib and dexamethasone respectively) were examined separately to determine if the model was significantly impacted by the specific therapeutic agent. As shown in **Figures 8C** and **8D****,** the high risk model successfully identified the patients at high risk of death in both groups (P=0.049, HR=2.3 and P=0.017, HR=2.5 respectively).

**Table 9**

| **List of genes comprising the 16-gene high-risk signature.** | | |
|---|---|---|
| Chromosome | Affymetrix Probe Set | Gene Symbol |
| 1q22 | 206513_at | *AIM2* |
| 21q22.3 | 211576_s_at | *SLC19A1* |
| 3p21.3 | 204016_at | *LARS2* |
| 1q31 | 219918_s_at | *ASPM* |
| 1p36.33-p36.21 | 213607_x_at | *FLJ13052* |
| 1q21.2 | 201897_s_at | *CKS1B* |
| 7p14-p13 | 220789_s_at | *TBRG4* |
| 1q43 | 242488_at | *NA* |
| 1pter-q31.3 | 206364_at | *KIF14* |
| 12q22 | 203432_at | *TMPO* |
| 8q23.1 | 200638_s_at | *YWHAZ* |
| 10q23.31 | 205235_s_at | *MPHOSPH1* |
| 1p13.2 | 200850_s_at | *AHCYL1* |
| 1p13.3 | 213628_at | *MCLC* |
| 1p22 | 218924_s_at | *CTBS* |
| 2p22-p21 | 202729_s_at | *LTBP1* |

**Table 10**

| **Confusion matrix of subgroup designations by the 17- and 16-gene models in the 144 cases.** | | | | |
|---|---|---|---|---|
| Model | 17 Gene | High-Risk (N=26) | Low-Risk (N=118) | Accuracy (%) |
| | High-Risk | 24 | 3 | |
| 16 Gene | Low-Risk | 2 | 115 | 96.5 |

The following references are cited herein:
- 1.: Smadja NV et al. Leukemia. 1998;12:960-969.
- 2.: Wuilleme S et al. Leukemia. 2002;19:275-278.
- 3.: Cremer FW et al. Genes Chromosomes Cancer. 2005;44:194-203.
- 4.: Gutierrez NC et al. Blood. 2004;104:2661-2666.
- 5.: Fonseca R et al. Cancer Res. 2004;64:1546-1558.
- 6.: Carrasco D et al. Cancer Cell. 2006;9:313-325.
- 7.: Shaughnessy J, Barlogie, B. Immunol. Rev. 2003;94:140-163.
- 8.: Kuehl WM, Bergsagel PL Nature Rev. Cancer. 2002;2:175-187.
- 9.: Zhan F et al. Blood. 2006;108:2020-2028.
- 10.: Avet-Loiseau et al. Genes Chromosomes Cancer. 1997;19:124-133.
- 11.: Sawyer JR et al. Blood. 1998;91:1732-1741.
- 12.: Le Baccon et al. Genes Chromosomes Cancer. 2001;32:250-64.
- 13.: Sawyer JR, et al. Genes Chromosomes Cancer. 2005;42:95-106.
- 14.: Rosinol L, Carrio A, Blade J, et al. Br J Haematol. 2005;130:729-732.
- 15.: Hanamura et al. Blood. 2006;16: Epub ahead of print.
- 16.: Zhan F, et al. Blood. 2002; 99:1745-1757.
- 17.: Storey JD, Tibshirani R. Proc Natl Acad Sci. 2003;100:9440-9445.
- 18.: Kaplan EL, Meier P,J Am Stat Assoc 1958; 53:457-481,
- 19.: Mantel N. Cancer Chemother Rep 1966; 50:163-170.
- 20.: O'Quigley J, Xu R, Stare J. Stat Med. 2005;24:479-489.
- 21.: R. Development Core Team. Vienna, Austria. 2004; (ISBM 3-900051-07-0, URL http://www.R-project.org).
- 22.: Rao CR., Wiley, New York (1973).
- 23.: Greipp P et al. J Clin Oncol. 2005;23:3412-20.
- 24.: Shaughnessy, J. et al. Blood. 2003;101:3849-3856.
- 25.: Kapanadze et al. FEBS Lett. 1998;426:266-270.
- 26.: Itoyama et al. Genes Chromosomes Cancer. 2002;35:318-328.
- 27.: Lu YJ et al. Lancet 2002;360:385-386.
- 28.: Hattinger et al. Br J Cancer. 2002;86:1763-1769.
- 29.: Cheng KW, et al. Nat Med. 2004; 10:1251-2156
- 30.: Zudaire I et al. Histopathology. 2002;40:547-555.
- 31.: Richardson P et al. N Engl J Med 2005; 16; 352: 2487-2498.
- 32.: Richardson P et al. Blood 2007 Aug 9; [epub ahead of print].
- 33.: Shaughnessy JD et al. Blood 2007; 109: 2276-2284.
- 34.: Mulligan G et al. Blood 2007; 109: 3177-3188.
- 35.: Shaffer AL et al. Immunity 2001; 15: 375-385.
- 36.: Ferrando AA et al. Cancer Cell 2002; 1: 75-87.
- 37.: Ross ME et al. Blood 2004; 104: 3679-3687.

## Claims

1. A method of gene expression profiling to identify genomic signatures linked to a prognosis of survival in a subject with symptomatic multiple myeloma or multiple myeloma, comprising:
determining the expression levels of genes consisting of *KIF14, SLC19A1, CKS1B, YWHAZ, MPHOSPH1,* TMPO, *NADK, LARS2, TBRG4, AIM2, ASPM, AHCYL1, CTBS, MCLC, LTBP1* and genes detectable with nucleic acids having the Affymetrix reference numbers 242488_at and 1557277_a_at on a U133Plus2.0 Affymetrix microarray, in a nucleic acid sample from plasma cells isolated from the subject; and
determining the presence of abnormal expression levels, thereby prognosing the subject.

2. The method of claim 1, wherein the abnormal expression profile is evaluated by a discriminant score from multivariate step-wise discriminant analysis (MSDA).

3. The method of claim 2, wherein the discriminant score is calculated with the formula: 200638_s_at x 0.283 - 1557277_a_at x 0.296-200850_s_at x 0.208 + 201897_s_at x 0.314 - 202729_s_at x 0.287 + 203432_at x 0.251 + 204016_at x 0.193 + 205235_s_at x 0.269 + 206364_at x 0.375 + 206513_at x 0.158 + 211576_s_at x 0.316 + 213607_at x 0.232 - 213628_at x 0.251 - 218924_s_at x 0.230-219918_s_at x 0.402 + 220789_s_at x 0.191 + 242488_at x 0.148 where probe sets 1557277_a_at and 242488_at are part of a U133Plus2.0 Affymetrix microarray, and, wherein the variables represent the value for the particular probe and a score above a predetermined threshold indicates a high risk prognosis.

4. The method according to any one of claims 1 to 3, **characterised in that** a high mean ratio of expression is indicative of a genomic signature associated with high-risk disease and a low mean ratio of expression is indicative of a genomic signature associated with a low-risk disease.

5. The method according to claim 4, **characterised in that** the genomic signature of high-risk disease correlates with shorter duration of complete remission, event free, early disease-related death or a combination thereof and said genomic signature of low-risk disease correlates with longer duration of complete remission, longer survival, a good prognosis or a combination thereof.

6. The method according to any one of claims 1 to 5, **characterised in that** the method predicts clinical outcome and survival of an individual, is effective in selecting treatment for an individual suffering from a disease, predicts post-treatment relapse risk and survival of an individual, correlates molecular classification of a disease with the genomic signature defining the risk groups, or a combination thereof.

7. The method according to claim 6, **characterised in that** the molecular classification is CD1 and correlates with high-risk multiple myeloma genomic signature.

8. The method according to claim 7, **characterised in that** the CD1 classification comprises increased expression of MMSET, MAF/MAFB, PROLIFERATION signatures or a combination thereof.

9. The method according to claim 6, **characterised in that** the molecular classification is CD2 and correlates with low-risk multiple myeloma genomic signature.

10. The method according to claim 9, **characterised in that** the CD2 classification comprises HYPERDIPLOIDY, LOW BONE DISEASE, CCND1/CCND3 translocations, CD20 expression or a combination thereof.

## Patentansprüche

1. Verfahren zur Genexpressions-Profilierung zur Identifizierung von Genomsignaturen in Verbindung mit einer Prognose des Überlebens in einem Subjekt mit symptomatischem Multiplem Myelom oder Multiplem Myelom, umfassend:
Bestimmung der Expressionsebenen von Genen, bestehend aus *KIF14, SLC19A1, CKS1B, YWHAZ, MPHOSPH1,* TMPO, *NADK, LARS2, TBRG4, AIM2, ASPM, AHCYL1, CTBS, MCLC, LTBP1* und mit Nukleinsäure nachweisbaren Genen mit den Affymetrix-Referenznummern 242488_at und 1557277_a_ar auf einem U133plus2.0 Affymetrix-Microarray, in einer Nukleinsäureprobe von Plasmazellen vom Subjekt isoliert; und
Bestimmung des Vorhandenseins von ungewöhnlichen Expressionsebenen, wobei das Subjekt prognostiziert wird.

2. Verfahren nach Anspruch 1, wobei das ungewöhnliche Expressionsprofil durch einen Diskriminanz-Score aus der multivariaten, stufenweisen Diskriminanzanalyse (MSDA) ausgewertet wird.

3. Verfahren nach Anspruch 2, wobei das Expressionsprofil berechnet wird anhand der Formel: 200638_s_at x 0,283 - 1557277_a_at x 0,296-200850_s_at x 0,208 + 201897_s_at x 0,314 - 202729_s_at x 0,287 + 203432_at x 0,251 + 204016_at x 0,193 + 205235_s_at x 0,269 + 206364_at x 0,375 + 206513_at x 0,158 + 211576_s_at x 0,316 + 213607_at x 0,232 - 213628_at x 0,251 - 218924_s_at x 0,230 - 219918_s_at x 0,402 + 220789_s_at x 0,191 + 242488_at x 0,148 wobei die Probesätze 1557277_a_at und 242488_at Teil eines U133Plus2.0 Affymetrix-Microarray sind, und wobei die Variablen den Wert für die einzelne Probe repräsentieren und ein Score über den vorgegebenen Grenzwert auf eine Hochrisikoprognose hinweist.

4. Verfahren nach einem der Ansprüche 1 bis 3, **dadurch gekennzeichnet, dass** eine hohe durchschnittliche Expressionsrate auf eine genomische Signatur in Verbindung mit einer Hochrisiko-Krankheit hindeutet und eine niedrige durchschnittliche Expressionsrate auf eine genomische Signatur in Verbindung mit einer Niedrigrisiko-Krankheit hindeutet.

5. Verfahren nach Anspruch 4, **dadurch gekennzeichnet, dass** die genomische Signatur einer Hochrisiko-Krankheit mit einer kürzeren Dauer der kompletten Remission, einem ereignisfreien, frühen krankheitsbezogenen Tod oder einer Kombination daraus korreliert, die genomische Signatur der Niedrigrisiko-Krankheit mit einer längeren Dauer der kompletten Remission, einem längeren Überleben, einer guten Prognose oder einer Kombination daraus korreliert.

6. Verfahren nach einem der Ansprüche 1 bis 5, **dadurch gekennzeichnet, dass** das Verfahren das klinische Ergebnis und das Überleben eines Individuums prognostiziert, effektiv ist bei der Auswahl der Behandlung für ein Individuum, das an einer Krankheit leidet, das Rückfallrisiko einer Nachbehandlung und das Überleben eines Individuums prognostiziert, die molekulare Klassifikation einer Krankheit mit der genomischen Signatur, die die Risikogruppen definiert, korreliert, oder eine Kombination daraus.

7. Verfahren nach Anspruch 6, **dadurch gekennzeichnet, dass** die molekulare Klassifikation CD1 ist und mit einer genomischen Signatur von Multiplem Myelom mit hohem Risiko korreliert.

8. Verfahren nach Anspruch 7, **dadurch gekennzeichnet, dass** die Klassifikation CD1 eine erhöhte Expression von MMSET, MAF/MAFB, PROLIFERATION Signaturen oder eine Kombination daraus umfasst.

9. Verfahren nach Anspruch 6, **dadurch gekennzeichnet, dass** die molekulare Klassifikation CD2 ist und mit einer genomischen Signatur von Multiplem Myelom mit niedrigem Risiko korreliert.

10. Verfahren nach Anspruch 9, **dadurch gekennzeichnet, dass** die Klassifikation CD2 HYPERDIPLOIDY, LOW BONE DISEASE, CCND1/CCND3 Translokationen, CD20 Expression oder eine Kombination daraus umfasst.

## Revendications

1. Procédé d'établissement d'un profil d'expression de gènes aux fins d'identification de signatures du génome, en liaison à un pronostic de survie chez un sujet souffrant de myélome multiple symptomatique, ou de myélome multiple, comportant :
la détermination des niveaux d'expression de gènes, à savoir, *KIF14, SLC19A1, CKS1B, YWHAZ, MPHOSPH1,* TMPO, *NADK, LARS2, TBRG4, AIM2, ASPM, AHCYL1, CTBS, MCLC, LTBP1,* de même que les gènes détectables à l'aide d'acides nucléiques, ayant les numéros de référence Affymetrix sont 242488_at et 1557277_a_at et implantés sur une biopuce Affymetrix U133Plus2.0, dans un échantillon d'acide nucléique extrait de cellules plasmatiques isolées sur le sujet ; et
la détermination de la présence de niveaux d'expression anormaux, et ce faisant, l'établissement d'un pronostic pour le sujet.

2. Procédé selon la revendication 1, **caractérisé en ce que** le profil d'expression anormale est évalué à l'aide d'un score discriminant, obtenu par une analyse de discrimination multivariée par étapes (MSDA).

3. Procédé selon la revendication 2, **caractérisé en ce que** le score discriminant est calculé à l'aide de la formule : 200638_s_at x 0,283 - 1557277_a_at x 0,296-200850_s_at x 0,208 + 201897_s_at x 0,314 - 202729_s_at x 0,287 + 203432_at x 0,251 + 204016_at x 0,193 + 205235_s_at x 0,269 + 206364_at x 0,375 +206513_at x 0,158 + 211576_s_at x 0,316 + 213607_at x 0,232-213628_at x 0,251 - 218924_s_at x 0,230 - 219918_s_at x 0,402 + 220789_s_at x 0,191 + 242488_at x 0,148, dans lequel les jeux de sonde 1557277_a_at et 242488_at appartiennent à la biopuce U133Plus2.0 d'Affymetrix et dans lequel les variables représentent la valeur de la sonde particulière et dans lequel, en outre, un score supérieur à un seuil prédéterminé est l'indicateur d'un pronostic de risque élevé.

4. Procédé selon l'une quelconque des revendications 1 à 3, **caractérisé en ce qu'**une valeur élevée du rapport moyen d'expression est l'indicateur d'une signature génomique associée à une maladie de haut risque et une valeur faible du rapport moyen d'expression est l'indicateur d'une signature génomique associée à une maladie de bas risque.

5. Procédé selon la revendication 4, **caractérisé en ce que** la signature du génome correspondant à la maladie de haut risque est en corrélation avec une rémission complète de durée plus courte, une mort précoce liée à la maladie, sans que survienne aucun épisode d'une nouvelle maladie, ou une combinaison de ces facteurs, et **en ce que** ladite signature du génome de la maladie de risque faible est en corrélation avec une durée plus longue de la rémission complète, une survie de plus longue durée, un pronostic favorable ou une combinaison de ces facteurs.

6. Procédé selon l'une quelconque des revendications 1 à 5, **caractérisé en ce que** le procédé prédit à la fois le résultat clinique et la survie d'un individu, produit un résultat effectif en matière de sélection du traitement destiné à un individu souffrant de maladie, prédit le risque de rechute postérieure au traitement, de même que la survie d'un individu, est en corrélation avec la classification moléculaire d'une maladie possédant la signature de génome définissant les groupes à risque, ou une combinaison de ces facteurs.

7. Procédé selon la revendication 6, **caractérisé en ce que** la classification moléculaire est CD1 et est en corrélation avec la signature du génome correspondant au myélome multiple à haut risque.

8. Procédé selon la revendication 7, **caractérisé en ce que** la classification CD1 comporte une expression accrue des signatures MMSET, MAF/MAFB, PROLIFERATION, ou une combinaison de ces facteurs.

9. Procédé selon la revendication 6, **caractérisé en ce que** la classification moléculaire est CD2 et est en corrélation avec la signature du génome du myélome multiple de faible risque.

10. Procédé selon la revendication 9, **caractérisé en ce que** la classification CD2 comporte HYPERDIPLOIDY, LOW BONE DISEASE, les translocations de CCND1/CCND3, l'expression de CD20 ou une combinaison de ces facteurs.
